# EUROPEAN PATENT SPECIFICATION

(11) **EP 3 887 823 B1**
(45) Date of publication and mention of the grant of the patent: **17.01.2024**
(21) Application number: 19806285.3
(22) Date of filing: 27.11.2019
(51) Int. Cl.: G01N 33/50

(54) **METHODS AND KIT FOR ASSAYING LYTIC POTENTIAL OF IMMUNE EFFECTOR CELLS**
VERFAHREN UND KIT ZUR UNTERSUCHUNG DES LYTISCHEN POTENZIALS VON IMMUNEFFEKTORZELLEN
PROCÉDÉS ET TROUSSE POUR TESTER LE POTENTIEL LYTIQUE DE CELLULES EFFECTRICES IMMUNITAIRES

(30) Priority: 28.11.2018 EP 18306576
(43) Date of publication of application: 06.10.2021
(73) Proprietor: Institut National de la Santé et de la Recherche Médicale (INSERM), 75013 Paris (FR); Université Paul Sabatier Toulouse III, 31400 Toulouse (FR)
(72) Inventor: VALITUTTI, Salvatore, 31037 Toulouse (FR); FILALI, Liza, 31037 Toulouse (FR); MUELLER, Sabina, 53717 Toulouse (FR); PUISSEGUR, Marie-Pierre, 31037 Toulouse Cedex 1 (FR)
(74) Representative: Inserm Transfert
(86) International application number: PCT/EP2019/082672
(87) International publication number: WO 2020/109355

(56) References cited:
- L. PIRIOU ET AL: "Design of a flow cytometric assay for the determination of natural killer and cytotoxic T-lymphocyte activity in human and in different animal species", CYTOMETRY, vol. 41, no. 4, 1 December 2000 (2000-12-01), pages 289-297, XP055371684, US ISSN: 0196-4763, DOI: 10.1002/1097-0320(20001201)41:4<289::AID-C YTO7>3.0.CO;2-5
- YAMAGUCHI YUKIE ET AL: "Natural killer cells control a T-helper 1 response in patients with BehÃ PRG et's disease", ARTHRITIS RESEARCH AND THERAPY, BIOMED CENTRAL, LONDON, GB, vol. 12, no. 3, 11 May 2010 (2010-05-11), page R80, XP021085208, ISSN: 1478-6354, DOI: 10.1186/AR3005
- FLIEGER D ET AL: "A novel non-radioactive cellular cytotoxicity test based on the differential assessment of living and killed target and effector cells", JOURNAL OF IMMUNOLOGICAL METHODS, ELSEVIER SCIENCE PUBLISHERS B.V.,AMSTERDAM, NL, vol. 180, no. 1, 13 March 1995 (1995-03-13), pages 1-13, XP004021065, ISSN: 0022-1759, DOI: 10.1016/0022-1759(94)00293-6
- YOHKO NAKAGAWA ET AL: "One-step simple assay to determine antigen-specific cytotoxic activities by single-color flow cytometry", BIOMEDICAL RESEARCH., vol. 32, no. 2, 1 January 2011 (2011-01-01), pages 159-166, XP055588506, JP ISSN: 0388-6107, DOI: 10.2220/biomedres.32.159

## Description

### FIELD:

The present disclosure is in the field of medicine.

### BACKGROUND:

It is generally accepted that CD8⁺ T cells, and in particular cytotoxic T lymphocytes (CTL), play an essential role in anti-tumor immune response. Accordingly, therapeutic protocols designed to potentiate CTL responses against tumor cells are frontline treatments of cancer patients (Chen and Mellman, 2017; Schumacher et al., 2015). Major needs in clinical oncology are to: i) better understand CTL biology in cancer patients; ii) improve monitoring of CTL effector function against tumors; iii) optimize immunotherapy protocols by identifying the parameters that influence the efficacy of CTL responses against tumor cells. Unfortunately, these needs are far from being fulfilled because of several technical and ethical constraints. The study of human CTL responses in clinical settings is indeed limited by the scarcity of cancer patients' derived samples and by the uniqueness of sample collections at the time of biopsy or of surgery. Those limitations make it difficult to obtain comprehensive results from experiments performed using patients' derived samples as it is routinely achieved with *in vitro* expanded cell lines or mouse models.

A key pathway used by human CTL to kill their target cells is based on perforin/granzyme-mediated lethal hit delivery. Within minutes or seconds after productive TCR engagement, the secretion of pore-forming protein perforin, granzyme B, and other proteases stored in CTL cytoplasmic granules (named lytic granules) takes place at the CTL/target cell lytic synapse (Baran et al., 2009; Bertrand et al., 2013; Faroudi et al., 2003; Law et al., 2010; Stinchcombe et al., 2001). Perforin-mediated penetration of granzyme B into target cells triggers an apoptotic cascade leading to target cell death (Lopez et al., 2013; de Saint Basile et al., 2010; Thiery et al., 2011). Recent works put forth the notion that lytic synapses are the privileged sites where the cytotoxic mechanisms of CTL are triggered and rapidly executed and where the first defense strategies of tumor cells are deployed (Bertrand et al., 2013; Khazen et al., 2016). The results unveil the existence of a real fight taking place on the two sides of the immunological synapse between CTL and tumor target cells. In particular, it was shown that melanoma cells are resistant to CTL-mediated cytotoxicity when compared to conventional cytotoxicity-sensitive target cells (Caramalho et al., 2009; Khazen et al., 2016). It was also shown that melanoma cells undergo a rapid Ca²⁺ dependent mechanism of membrane reparation that occurs within seconds after initial productive TCR engagement and is triggered by perforin-mediated pore formation in target cell plasma membrane (Khazen et al., 2016). Those results are in line with the hypothesis that melanoma cells might have hijacked ancestral membrane repair mechanisms to resist to CTL attack. It is indeed well established that upon plasma membrane injury, cells undergo a calcium dependent lysosome docking and fusion to the plasma membrane that is accompanied by a membrane reparative turnover (Andrews et al., 2014; Cheng et al., 2015; Horn and Jaiswal, 2018).

L.Piriou et al. disclose that a flow cytometric assay using a lipophilic carbocyanine dye is highly reproducible and is suitable to measure different types of cell cytotoxicity (Piriou, L et al. "Design of a flow cytometric assay for the determination of natural killer and cytotoxic T-lymphocyte activity in human and in different animal species." Cytometry vol. 41,4 (2000): 289-97).

Y. Yamaguchi et al. disclose an assay for determining the cytotoxic activity of natural killer cells, wherein target cells are pre-stained with a lipophilic green fluorescent membrane dye and dead cells are detected by incubation with a DNA staining solution and subsequent analysis is performed on a flow cytometer (Yamaguchi, Yukie et al. "Natural killer cells control a T-helper 1 response in patients with Behcet's disease." Arthritis research & therapy vol. 12,3 (2010): R80).

D. Flieger et al. disclose a non-radioactive flow cytometric assay for the quantitative analysis of cell-mediated cytotoxicity, wherein prior to incubation the effector cells are stained with a red lipophilic fluorescent dye PKH26 and the target cells (e.g. cancer cells) with a green fluorescent dye PKH2 (Flieger, D et al. "A novel non-radioactive cellular cytotoxicity test based on the differential assessment of living and killed target and effector cells." Journal of immunological methods vol. 180,1 (1995): 1-13).

Y.Nakagawa et al. disclose a flow cytometric assay to asses antigen-specific cytotoxic activities of cytotoxic T-cell lymphocytes *in vitro* using single fluorescent-dye 5-carboxyfluorescein diacetate succinimydyl ester (CFSE)(Nakagawa, Yohko et al. "One-step simple assay to determine antigen-specific cytotoxic activities by single-color flow cytometry." Biomedical research (Tokyo, Japan) vol. 32,2 (2011): 159-66).

### SUMMARY:

The present invention is defined by the claims. In particular, the present invention relates to a method of assaying the lytic potential of a population of immune effector cells comprising the steps consisting of i) contacting the population of immune effector cells with a population of target cells for a sufficient period of time and under conditions suitable for allowing the population of immune effector cells to induce a cytotoxic response, ii) measuring the reparative membrane turnover level in the population of target cells by measuring the uptake of a fluorescent lipophilic dye by the population of target cells, wherein said level correlates with the lytic potential of the population of immune effector cells and wherein the fluorescent lipophilic dye is a styryl dye.

The following detailed description, figures and example do not fall under the scope of the present invention and are present for understanding and illustration purposes only. Furthermore, any reference in the description to methods of treatment refers to the compounds, pharmaceutical compositions and medicaments of the present invention for use in a method of treatment of the human body by therapy.

### DETAILED DESCRIPTION:

To overcome the difficulty in achieving a quantitative assessment of CTL function in clinical settings, we aimed at developing a new method inspired by our knowledge of the CTL/tumor target biology and based on flow cytometry. The methodology allows to rapidly assessing the synaptic resistance of tumor target cells to CTL attack and the intrinsic capacity of CD8⁺CTL to efficiently kill their target cells.

Accordingly, the first object of the present disclosure relates to a method of assaying the lytic potential of a population of immune effector cells comprising the steps consisting of i) contacting the population of immune effector cells with a population of target cells for a sufficient period of time and under conditions suitable for allowing the population of immune effector cells to induce a cytotoxic response, ii) measuring the reparative membrane turnover level in the population of target cells by measuring the uptake of a fluorescent lipophilic dye by the population of target cells, wherein said level correlates with the lytic potential of the population of immune effector cells and wherein the fluorescent lipophilic dye is a styryl dye.

As used herein, the term "immune effector cell" or "cytotoxic effector cell" refers to a cell that is capable of killing or directly or indirectly bringing about the death of a target cell (i.e. "lytic potential") displaying an antigen against which the effector cell is directed. Preferred effector cells use perforin for killing the target cells and include, but are not limited to cytotoxic T lymphocytes (CTLs), natural killer (NK) cells, and natural killer T (NKT) cells. In particular, the population of immune effector cells is a population of cytotoxic T lymphocytes. As used herein, the term "cytotoxic T lymphocyte" or "CTL" has its general meaning in the art and refers to a subset of T cells that express CD8 on their surface and contain lytic granules. CD8 antigens are members of the immunoglobulin supergene family and are associative recognition elements in major histocompatibility complex class I-restricted interactions. They are MHC class I-restricted, and function as cytotoxic T cells. Cytotoxic T lymphocytes are also called, CD8+ T cells, T-killer cells, cytolytic T cells, or killer T cells.

In particular, the population of immune effector cells is a population of tumor infiltrating cytotoxic T lymphocytes. As used herein, the term "tumor infiltrating cytotoxic T lymphocyte" or "TIL" refers to the pool of cytotoxic T lymphocytes of the patient that have left the blood stream and have migrated into a tumor.

As used herein, the term "target cell" has its general meaning in the art and refers to a cell against which the activity of a cytotoxic effector cell is tested. Preferred target cells can display one or more than one antigen, more preferably in a MHC-I restricted manner. Preferably, target cells are tumor cells, or cells infected (naturally or not) by a pathogen (virus, bacterium, parasite...). In particular, the target cells are EBV-transformed cells (e.g. JY cells).

In particular, the target cells are previously prepared before being contacted by the population of immune effector cells. For instance, in particular, it may be beneficial or desirable to pulse the target cells with at least one antigen. The antigen comprises "epitope" which consist of portion of the antigen that are recognized by the immune effector cells. For example, interaction of such epitope with an antigen recognition site of a T cell antigen receptor (TCR) leads to the induction of antigen-specific immune response (i.e. cytotoxic attack). Typically, said antigen is a peptide. In particular, the target cells are pulsed with a peptide corresponding to the amino acid sequence of an infectious agent or a tumor antigen. As used herein, the term "tumor antigen" includes both tumor specific antigen (TSA) and tumor associated antigen (TAA). A tumor specific antigen is known as an antigen that is expressed only by tumor cells while tumor associated antigen are expressed on tumor cells but may also be expressed on some normal cells. Tumor specific antigens and tumor associated antigens have been described in the art. Such tumor antigen can be, but is not limited to human epithelial cell mucin (Muc-1; a 20 amino acid core repeat for Muc-1 glycoprotein, present on breast cancer cells and pancreatic cancer cells), the Ha-ras oncogene product, p53, carcino-embryonic antigen (CEA), the raf oncogene product, GD2, GD3, GM2, TF, sTn, MAGE-1, MAGE-3, tyrosinase, gp75, Melan-A/Mart-1, gp100, HER2/neu, EBV-LMP 1 & 2, HPV-F4, 6, 7, prostatic serum antigen (PSA), alpha-fetoprotein (AFP), CO17-1A, GA733, gp72, p53, the ras oncogene product, proteinase 3, Wilm's tumor antigen-1, telomerase, HPV E7 and melanoma gangliosides, as well as any other tumor antigens now known or identified in the future. Other antigenic determinant include without limitation, antigens of parasite or fungus (such as candida, trichophyton), bacterial cell (e.g staphylococcus, pneumoccus or streptococcus cell, Borrelia, pseudomonas, listeria), viral particle (e.g. HIV, HBV, HPV, HSV, HVT, CMV, HTLV, hepatitis C virus, rotavirus, flavivirus, rous associated virus, or SARS virus, yellow fever virus or dengue virus), or any portion thereof.

In particular, the population of cells (i.e. immune effector cells or target cells) are typically primary cells or cell lines.

In particular, the population of cells (i.e. immune effector cells or target cells) is isolated from a biological sample obtained from a patient.

As used herein, the term "sample" to any biological sample obtained from the purpose of evaluation in vitro. The sample is typically a tissue sample or a body fluid sample. The term "tissue sample" includes sections of tissues such as biopsy or autopsy samples and frozen sections taken for histological purposes. In particular, the tissue sample is a tumor tissue sample. The term "tumor tissue sample" means any tissue tumor sample derived from the patient. Said tissue sample is obtained for the purpose of the in vitro evaluation. In particular, the tumor sample may result from the tumor resected from the patient. In particular, the tumor sample may result from a biopsy performed in the primary tumour of the patient or performed in metastatic sample distant from the primary tumor of the patient. Examples of body fluids are blood, serum, plasma, amniotic fluid, brain/spinal cord fluid, liquor, cerebrospinal fluid, sputum, throat and pharynx secretions and other mucous membrane secretions, synovial fluids, ascites, tear fluid, lymph fluid and urine. More particularly, the sample is a blood sample. As used herein, the term "blood sample" means a whole blood sample obtained from the patient.

In particular, the population of target cells is a population of tumor cells.

As used herein, the term "contacting" to a population of immune effector cells and/or a population of target cells refers to placing the population of immune effector cells and/or the population of target cells into a buffer and/or medium wherein the cells are capable of interacting (e.g. inducing a cytotoxic response). Typically the population of immune effector cells and the population of target cells are contacted for a period of 2, 5, 10, 15, 30 or 60 minutes. Any culture medium suitable for growth, survival and differentiation of immune effector cells may be used. Typically, it consists of a base medium containing nutrients (a source of carbon, aminoacids), a pH buffer and salts, which can be supplemented with serum of human or other origin and/or growth factors and/or antibiotics. Typically, the base medium can be RPMI 1640, DMEM, IMDM, X-VIVO or AIM-V medium, all of which are commercially available standard media. Typically the ratio between the population of immune effector cells and the population of target cells is 2:1.

In particular, the method of the present disclosure further comprises the step consisting of measuring membrane turnover level in the population of immune effector cells. Accordingly, in particular, membrane turnover level of target cells and the membrane turnover level of immune effector cells both are both measured. In particular, the ratio between both membrane turnover levels indicates the lytic potential of the immune effector cells.

Measuring the reparative membrane turnover level may be determined by any assay well known in the art. Typically, the level is determined by using a fluorescent lipophilic dye.

In particular, the method further comprises the step of measuring the uptake of the fluorescent lipophilic dye by the population of immune effector cells.

As used herein, the term "styryl dye" has its general meaning in the art and refers to a dye having a structure in which a hetero atom having a positive charge and a carbon ring-type aromatic ring are bonded by a dimethine chain or a polymethine chain. Preferred styryl dyes include, but are not limited to FM1-43, FM4-64, FM14-68, FM2-10, FM4-84, FM1-84, FM14-27, FM14-29, FM3-25, FM3-14, FM5-55, RH414, FM6-55, FM10-75, FM1-81, FM9-49, FM4-95, FM4-59, FM9-40, and combinations thereof. Preferred dyes such as FM1-43 are only weakly fluorescent in water but very fluorescent when associated with a membrane, such that dye uptake is readily discernable. Suitable dyes are available commercially, i.e., Molecular Probes, Inc., of Eugene, Oreg., "Handbook of Fluorescent Probes and Research Chemicals", 6th Edition, 1996, particularly, Chapter 17, and more particularly, Section 2 of Chapter 17, (including referenced related chapter).

In general, the fluorescent lipophilic dye is provided to the cells at a concentration ranging from about 5µg/ml to about 20µg/ml, preferably 10µg/ml. A wash step may or may not be used.

The fluorescent dye uptake is measured using devices that measure cell fluorescence, such as a Fluorescence Activated Cell Sorter (FACS) machine. As used herein, the term "fluorescence activated cell sorting" or "FACS" refers to a method by which the individual cells of a sample are analyzed and sorted according to their optical properties (e.g., light absorbance, light scattering and fluorescence properties, etc.) as they pass in a narrow stream in single file through a laser beam. Fluorescence-activated cell sorting is a specialized type of flow cytometry. It provides a method for sorting a heterogeneous mixture of biological cells into two or more containers, one cell at a time, based upon the specific light scattering and fluorescent characteristics of each cell. It is a useful scientific instrument as it provides fast, objective and quantitative recording of fluorescent signals from individual cells as well as physical separation of cells of particular interest. In a typical FACS system, the cell suspension is entrained in the center of a narrow, rapidly flowing stream of liquid. The flow is arranged so that there is a large separation between cells relative to their diameter. A vibrating mechanism causes the stream of cells to break into individual droplets. The system is adjusted so that there is a low probability of more than one cell being in a droplet. Just before the stream breaks into droplets the flow passes through a fluorescence measuring station where the fluorescent character of interest of each cell is measured. An electrical charging ring is placed just at the point where the stream breaks into droplets. A charge is placed on the ring based on the immediately prior fluorescence intensity measurement and the opposite charge is trapped on the droplet as it breaks from the stream. The charged droplets then fall through an electrostatic deflection system that diverts droplets into containers based upon their charge. In some systems the charge is applied directly to the stream and the droplet breaking off retains charge of the same sign as the stream. The stream is then returned to neutral after the droplet breaks off. The fluorescent labels for FACS technique depend on the lamp or laser used to excite the fluorescent dye and on the detectors available. The most commonly available lasers on single laser machines are blue argon lasers (488 nm).

In particular, FACS brings the advantage that by using a gating strategy it is possible in the same sample to determine the fluorescent dye uptake in both population of cells (i.e. target cells and immune effector cells). For instance, one cell type can be loaded with a fluorescent probe to distinguish the two cell types. A useful probe is the marker of proliferation CellTrace violet that has a fluorescence emission that does not overlap with that of FM1-43 and is not toxic for cells. In this context this marker is not used to measure cell proliferation, but to stain one cell type.

In particular, the fluorescent dye uptake is expressed as an absolute value (e.g. fluorescence intensity) or as a rate (e.g. fluorescence intensity by a section of time). In particular, the fluorescence intensity is expressed as MFI. The term "MFI", as used herein, refers to the mean or median fluorescence intensity of a population of fluorescent cells. In particular, the fluorescent uptake is expressed as a rate that consists of measuring the MFI per minute.

In particular, the method of the present disclosure comprises the step consisting of comparing the reparative membrane turnover level (e.g. fluorescent dye uptake) to a reference value. Typically said reference value may corresponds to the reparative membrane turnover level (e.g. the fluorescent uptake) measured in target cells that are not contacted by the immune effector cells. In particular, the reference value is the reparative membrane turnover level (e.g. the fluorescent uptake) measured in control target cells that are contacted with the immune effector cells. For instance, said control target cells are typically cell lines, or e EBV transformed cells (e.g. JY cells).

In particular, the fluorescent dye uptake is measured in combination with at least one other parameter, such as cell death of the target cells that can be measured by any assay well known in the art (e.g. use of a viability dye (e.g. eFluor780), measuring apoptosis by annexin V).

The method of the present disclosure may find uses in a wide number of contexts.

For example, the method can be used to screen for the ability of a test agent (e.g. a peptide, a small organic molecule, a vaccine, a nucleic acid, etc.) to induce a class I-restricted cell-mediated cytotoxicity directed against a particular antigen. This method would involve administering to the subject organism the test agent, obtaining an effector cell from the organism; and measuring cytotoxic activity of the effector cell against a target displaying the antigen. In particular, the method of the present disclosure is used to determine if a subject has been exposed to (or is presently exposed to) one or more particular antigens. For instance, the method of the disclosure can be used to see if a subject has any immunity left from previous vaccinations/immunizations. Known antigens associated with a given vaccine, for example, can be used to detect and quantitate any effector cells present in a biological sample obtained from the subject. One can also use the method of the present disclosure to identify the best antigen or combinations of antigens for a particular vaccine (e.g. for a particular year's influenza vaccine). In particular, the method of the present disclosure is also particular suitable for optimizing an antigen for use in a vaccine. The method typically involves providing a plurality of antigens that are candidates for the vaccine; screening the antigens using any of the methods and the disclosure; and selecting an antigen that is capable of increasing the lytic potential of the immune effector cells.

The method of the present disclosure is also particularly suitable for detecting the presence of memory cytotoxic effector activity.

In particular, the method of the present disclosure can be used to determine if a subject would reject transplant, wherein the immune effector cells of the recipient are contacted with target cells that come from the donor.

In particular, the method of the present disclosure is particularly suitable for diagnosing autoimmune diseases, and more particularly for identifying the nature of the auto antigen.

In particular, the method of the present disclosure is particularly suitable for assessing the resistance of some target cells (e.g. tumor cells) to a cytotoxic response. In particular, the method of the present disclosure is suitable for assessing the magnitude of target cell synaptic defence to perforation. Typically, freshly isolated target cells (e.g. tumor cells) from a biological obtained from a patient, optionally pulsed an antigenic determinant, are contacted with the immune effector. Then the reparative membrane turnover level is measured and indicates the resistance of the target cells to the cytotoxic responses. Typically an increased membrane turnover level (e.g. in comparison with control target cells such as EBV-transformed cells (e.g. JY cells)) indicates that said target cells are resistant to the cytotoxic response.

In particular, the method of the present disclosure is particularly suitable for screening a test compound for the ability to modulate (i.e. increase or decrease) the lytic potential of a population of immune effector cells.

Accordingly a further object relates to a method of screening a test compound for the ability to modulate (i.e. increase or decrease) the lytic potential of a population of immune effector cells comprising the steps consisting of i) contacting the population of immune effector cells with a population of target cells for a sufficient period of time and under conditions suitable for allowing the population of immune effector cells to induce a cytotoxic response in presence of the test compound, ii) measuring the reparative membrane turnover level in the population of target cells by measuring the uptake of a fluorescent lipophilic dye by the population of target cells, iii) comparing the reparative membrane turnover level determined at step ii) with the reparative membrane turnover level measured in absence of the test compound and iv) selecting the test compound wherein a difference is detected between the reparative membrane turnover level determined at step ii) and the reparative membrane turnover level measured in absence of the test compound, wherein the fluorescent lipophilic dye is a styryl dye.

The term "test compound" refers generally to a material that is expected to increase, decrease, reduce, suppress or inhibit reparative membrane turnover. Typically, the test compound includes small molecules, high molecular weight molecules, mixture of compounds such as natural extracts or cell or tissue culture products, biological material such as proteins, antibodies, peptides, DNA, RNA, antisense oligonucleotides, RNAi, aptamer, RNAzymes and DNAzymes, or glucose and lipids, but is not limited thereto. The test compounds may be polypeptides having amino acid residues of below 20, particularly 6, 10, 12, 20 aa or above 20 such as 50aa. These materials are obtained from synthetic or natural compound libraries and the methods to obtain or construct libraries are known in the art. For example, synthetic chemical library may be obtained from Maybridge Chemical Co.(UK), Comgenex(USA), Brandon Asociates(USA), Microsource(USA) and Sigma-Aldrich(USA). The chemical library of natural origin may be obtained from Pan Laboratories (USA) and MycoSearch(USA). Further test compounds may be obtained by various combinatorial library construction methods known in the art including for example, biological libraries, spatially addressable parallel solid phase or solution phase libraries. Test compound of a library may be composed of peptides, peptoides, circular or liner oligomeric compounds, template based compounds such as benzodiazepine, hydantoin, biaryls, carbocyclic and polycyclic compounds such as naphthalene, phenothiazine, acridine, steroids and the like, carbohydrate and amino acid derivatives, dihydropyridine, benzhydryl and heterocyclic compounds such as triazine, indole, thiazolidine and the like, but does not limited thereto.

In particular, the test compound has been previously selected to modulate the activity or expression of an immune checkpoint protein. As used herein the term "immune checkpoint protein" has its general meaning in the art and refers to a molecule that is expressed by T cells in that either turn up a signal (stimulatory checkpoint molecules) or turn down a signal (inhibitory checkpoint molecules). Immune checkpoint molecules are recognized in the art to constitute immune checkpoint pathways similar to the CTLA-4 and PD-1 dependent pathways (see e.g. Pardoll, 2012. Nature Rev Cancer 12:252-264; Mellman et al., 2011. Nature 480:480-489). Examples of inhibitory checkpoint molecules include B7-H3, B7-H4, BTLA, CTLA-4, CD277, KIR, PD-1, LAG-3, TIM-3, TIGIT and VISTA. B7-H3, also called CD276, was originally understood to be a co-stimulatory molecule but is now regarded as co-inhibitory. B7-H4, also called VTCN1, is expressed by tumor cells and tumor-associated macrophages and plays a role in tumor escape. B and T Lymphocyte Attenuator (BTLA), also called CD272, is a ligand of HVEM (Herpesvirus Entry Mediator). Cell surface expression of BTLA is gradually downregulated during differentiation of human CD8+ T cells from the naive to effector cell phenotype, however tumor-specific human CD8+ T cells express high levels of BTLA. CTLA-4, Cytotoxic T-Lymphocyte-Associated protein 4 and also called CD152 is overexpressed on Treg cells serves to control T cell proliferation. KIR, Killer-cell Immunoglobulin-like Receptor, is a receptor for MHC Class I molecules on Natural Killer cells. LAG3, Lymphocyte Activation Gene-3, works to suppress an immune response by action to Tregs as well as direct effects on CD8+ T cells. TIM-3, short for T-cell Immunoglobulin domain and Mucin domain 3, expresses on activated human CD4+ T cells and regulates Th1 and Th17 cytokines. TIM-3 acts as a negative regulator of Th1/Tc1 function by triggering cell death upon interaction with its ligand, galectin-9. VISTA, short for V-domain Ig suppressor of T cell activation, is primarily expressed on hematopoietic cells so that consistent expression of VISTA on leukocytes within tumors may allow VISTA blockade to be effective across a broad range of solid tumors. As used herein, the term "PD-1" has its general meaning in the art and refers to programmed cell death protein 1 (also known as CD279). PD-1 acts as an immune checkpoint, which upon binding of one of its ligands, PD-L1 or PD-L2, enables Shp2 to dephosphorylate CD28 and inhibits the activation of T cells.

In particular, the method of the present disclosure is particularly suitable for detecting T cell exhaustion in a patient. Typically, a population of immune effector cells (i.e. freshly Tumor Infiltrating Lymphocytes (TILs)) is isolated from a biological sample obtained from the patient and is used in the assay.

As used herein, the term "T cell exhaustion" refers to a state of T cell dysfunction. The T cell exhaustion generally arises during many chronic infections and cancer. T cell exhaustion can be defined by poor effector function, sustained expression of inhibitory receptors, and/or a transcriptional state distinct from that of functional effector or memory T cells. T cell exhaustion generally prevents optimal control of infection and tumors. See, e.g., Wherry E J, Nat Immunol. (2011) 12: 492-499, for additional information about T cell exhaustion. Typically, T cell exhaustion results from the binding of an immune checkpoint protein to at least one of its ligands (e.g. PD1-1 and one of its ligands PD-L1 or PD-L2).

In particular, the method of the present disclosure is particularly suitable for determining a patient suffering from a cancer will achieve a response with an immune checkpoint blockade therapy.

As used herein, the term "cancer" has its general meaning in the art and includes, but is not limited to, solid tumors and blood-borne tumors. The term cancer includes diseases of the skin, tissues, organs, bone, cartilage, blood and vessels. The term "cancer" further encompasses both primary and metastatic cancers. Examples of cancers that may be treated by methods and compositions of the disclosure include, but are not limited to, cancer cells from the bladder, blood, bone, bone marrow, brain, breast, colon, esophagus, gastrointestinal tract, gum, head, kidney, liver, lung, nasopharynx, neck, ovary, prostate, skin, stomach, testis, tongue, or uterus. In addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous; adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; Paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; and roblastoma, malignant; Sertoli cell carcinoma; Leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; malignant melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; Ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; Hodgkin's disease; Hodgkin's lymphoma; paragranuloma; malignant lymphoma, small lymphocytic; malignant lymphoma, large cell, diffuse; malignant lymphoma, follicular; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia.

Accordingly, a further object of the present disclosure relates to a method of determining whether a patient suffering from cancer will achieve a response with an immune checkpoint inhibitor, comprising the steps consisting of i) isolating a population of immune effector cells (e.g. TILs) from a biological sample obtained from the patient (e.g. tumor tissue sample or blood sample), ii) contacting the population of immune effector cells with a population of target cells for a sufficient period of time and under conditions suitable for allowing the population of immune effector cells to induce a cytotoxic response in presence of the immune checkpoint inhibitor, iii) measuring the reparative membrane turnover level in the population of target cells by measuring the uptake of a fluorescent lipophilic dye by the population of target cells, iv) comparing the reparative membrane turnover level determined at step iii) with the reparative membrane turnover level measured in absence of the immune checkpoint inhibitor and v) concluding that the patient will achieve a response with the immune checkpoint inhibitor when the reparative membrane turnover level determined at step iii) is higher than the reparative membrane turnover level measured in absence of the immune checkpoint inhibitor, wherein the fluorescent lipophilic dye is a styryl dye.

The method is thus particularly suitable for discriminating responder from non-responder. As used herein the term "responder" in the context of the present disclosure refers to a patient that will achieve a response, i.e. a patient where the cancer is eradicated, reduced or improved. According to the disclosure, the responders have an objective response and therefore the term does not encompass patients having a stabilized cancer such that the disease is not progressing after the immune checkpoint therapy. A non-responder or refractory patient includes patients for whom the cancer does not show reduction or improvement after the immune checkpoint therapy. According to the disclosure the term "non-responder" also includes patients having a stabilized cancer. Typically, the characterization of the patient as a responder or non-responder can be performed by reference to a standard or a training set. The standard may be the profile of a patient who is known to be a responder or non-responder or alternatively may be a numerical value. Such predetermined standards may be provided in any suitable form, such as a printed list or diagram, computer software program, or other media. When it is concluded that the patient is a non-responder, the physician could take the decision to stop the immune checkpoint therapy to avoid any further adverse sides effects.

Examples of immune checkpoint inhibitor includes PD-1 antagonists, PD-L1 antagonists, PD-L2 antagonists, CTLA-4 antagonists, VISTA antagonists, TIM-3 antagonists, LAG-3 antagonists, IDO antagonists, KIR2D antagonists, A2AR antagonists, B7-H3 antagonists, B7-H4 antagonists, and BTLA antagonists.

In particular, PD-1 (Programmed Death-1) axis antagonists include PD-1 antagonist (for example anti-PD-1 antibody), PD-L1 (Programmed Death Ligand-1) antagonist (for example anti-PD-L1 antibody) and PD-L2 (Programmed Death Ligand-2) antagonist (for example anti-PD-L2 antibody). In particular, the anti-PD-1 antibody is selected from the group consisting of MDX-1106 (also known as Nivolumab, MDX-1106-04, ONO-4538, BMS-936558, and Opdivo^{®}), Merck 3475 (also known as Pembrolizumab, MK-3475, Lambrolizumab, Keytruda^{®}, and SCH-900475), and CT-011 (also known as Pidilizumab, hBAT, and hBAT-1). In particular, the PD-1 binding antagonist is AMP-224 (also known as B7-DCIg). In particular, the anti-PD-L1 antibody is selected from the group consisting of YW243.55.S70, MPDL3280A, MDX-1105, and MEDI4736. MDX-1105, also known as BMS-936559, is an anti-PD-L1 antibody described in WO2007/005874. Antibody YW243.55. S70 is an anti-PD-L1 described in WO 2010/077634 A1. MEDI4736 is an anti-PD-L1 antibody described in WO2011/066389 and US2013/034559. MDX-1106, also known as MDX-1106-04, ONO-4538 or BMS-936558, is an anti-PD-1 antibody described in U.S. Pat. No. 8,008,449 and WO2006/121168. Merck 3745, also known as MK-3475 or SCH-900475, is an anti-PD-1 antibody described in U.S. Pat. No. 8,345,509 and WO2009/114335. CT-011 (Pidizilumab), also known as hBAT or hBAT-1, is an anti-PD-1 antibody described in WO2009/101611. AMP-224, also known as B7-DCIg, is a PD-L2-Fc fusion soluble receptor described in WO2010/027827 and WO2011/066342. Atezolimumab is an anti-PD-L1 antibody described in U.S. Pat. No. 8,217,149. Avelumab is an anti-PD-L1 antibody described in US 20140341917. CA-170 is a PD-1 antagonist described in WO2015033301 & WO2015033299. Other anti-PD-1 antibodies are disclosed in U.S. Pat. No. 8,609,089, US 2010028330, and/or US 20120114649. In particular, the PD-1 inhibitor is an anti-PD-1 antibody chosen from Nivolumab, Pembrolizumab or Pidilizumab. In particular, PD-L1 antagonist is selected from the group comprising of Avelumab, BMS-936559, CA-170, Durvalumab, MCLA-145, SP142, STI-A1011, STIA1012, STI-A1010, STI-A1014, A110, KY1003 and Atezolimumab and the preferred one is Avelumab, Durvalumab or Atezolimumab.

In particular, CTLA-4 (Cytotoxic T-Lymphocyte Antigen-4) antagonists are selected from the group consisting of anti-CTLA-4 antibodies, human anti-CTLA-4 antibodies, mouse anti-CTLA-4 antibodies, mammalian anti-CTLA-4 antibodies, humanized anti-CTLA-4 antibodies, monoclonal anti-CTLA-4 antibodies, polyclonal anti-CTLA-4 antibodies, chimeric anti-CTLA-4 antibodies, MDX-010 (Ipilimumab), Tremelimumab, anti-CD28 antibodies, anti-CTLA-4 adnectins, anti-CTLA-4 domain antibodies, single chain anti-CTLA-4 fragments, heavy chain anti-CTLA-4 fragments, light chain anti-CTLA-4 fragments, inhibitors of CTLA-4 that agonize the co-stimulatory pathway, the antibodies disclosed in PCT Publication No. WO 2001/014424, the antibodies disclosed in PCT Publication No. WO 2004/035607, the antibodies disclosed in U.S. Publication No. 2005/0201994, and the antibodies disclosed in granted European Patent No. EP 1212422 B. Additional CTLA-4 antibodies are described in U.S. Pat. Nos. 5,811,097; 5,855,887; 6,051,227; and 6,984,720; in PCT Publication Nos. WO 01/14424 and WO 00/37504; and in U.S. Publication Nos. 2002/0039581 and 2002/086014. Other anti-CTLA-4 antibodies that can be used in a method of the present disclosure include, for example, those disclosed in: WO 98/42752; U.S. Pat. Nos. 6,682,736 and 6,207,156; Hurwitz et al., Proc. Natl. Acad. Sci. USA, 95(17): 10067-10071 (1998); Camacho et al., J. Clin: Oncology, 22(145): Abstract No. 2505 (2004) (antibody CP-675206); Mokyr et al., Cancer Res., 58:5301-5304 (1998), and U.S. Pat. Nos. 5,977,318, 6,682,736, 7,109,003, and 7,132,281. A preferred clinical CTLA-4 antibody is human monoclonal antibody (also referred to as MDX-010 and Ipilimumab with CAS No. 477202-00-9 and available from Medarex, Inc., Bloomsbury, N.J.) is disclosed in WO 01/14424. With regard to CTLA-4 antagonist (antibodies), these are known and include Tremelimumab (CP-675,206) and Ipilimumab.

Other immune-checkpoint inhibitors include lymphocyte activation gene-3 (LAG-3) inhibitors, such as IMP321, a soluble Ig fusion protein (Brignone et al., 2007, J. Immunol. 179:4202-4211). Other immune-checkpoint inhibitors include B7 inhibitors, such as B7-H3 and B7-H4 inhibitors. In particular, the anti-B7-H3 antibody MGA271 (Loo et al., 2012, Clin. Cancer Res. July 15 (18) 3834). Also included are TIM-3 (T-cell immunoglobulin domain and mucin domain 3) inhibitors (Fourcade et al., 2010, J. Exp. Med. 207:2175-86 and Sakuishi et al., 2010, J. Exp. Med. 207:2187-94). As used herein, the term "TIM-3" has its general meaning in the art and refers to T cell immunoglobulin and mucin domain-containing molecule 3. The natural ligand of TIM-3 is galectin 9 (Gal9). Accordingly, the term "TIM-3 inhibitor" as used herein refers to a compound, substance or composition that can inhibit the function of TIM-3. For example, the inhibitor can inhibit the expression or activity of TIM-3, modulate or block the TIM-3 signalling pathway and/or block the binding of TIM-3 to galectin-9. Antibodies having specificity for TIM-3 are well known in the art and typically those described in WO2011155607, WO2013006490 and WO2010117057.

In particular, the immune checkpoint inhibitor is an IDO inhibitor. Examples of IDO inhibitors are described in WO 2014150677. Examples of IDO inhibitors include without limitation 1-methyl-tryptophan (IMT), β- (3-benzofuranyl)-alanine, β-(3-benzo(b)thienyl)-alanine), 6-nitro-tryptophan, 6- fluoro-tryptophan, 4-methyl-tryptophan, 5 -methyl tryptophan, 6-methyl-tryptophan, 5-methoxy-tryptophan, 5 -hydroxy-tryptophan, indole 3-carbinol, 3,3'-diindolylmethane, epigallocatechin gallate, 5-Br-4-Cl-indoxyl 1,3-diacetate, 9- vinylcarbazole, acemetacin, 5-bromo-tryptophan, 5-bromoindoxyl diacetate, 3- Amino-naphtoic acid, pyrrolidine dithiocarbamate, 4-phenylimidazole a brassinin derivative, a thiohydantoin derivative, a β-carboline derivative or a brassilexin derivative. Preferably the IDO inhibitor is selected from 1-methyl-tryptophan, β-(3- benzofuranyl)-alanine, 6-nitro-L-tryptophan, 3-Amino-naphtoic acid and β-[3- benzo(b)thienyl] -alanine or a derivative or prodrug thereof.

In particular, the method of the present disclosure is particularly suitable for stratifying patients suffering from cancer on the basis of a score that combines the ability of their CTL to provide a cytotoxic response and the ability of the tumor cells to resist to the cytotoxic response. In particular, the score is particular suitable for determining whether the patients will achieve a response to a particular treatment (e.g. immune checkpoint blockade therapy).

Accordingly, a further object of the present disclosure relates to a method of determining whether a patient suffering from a cancer will achieve a response with a treatment comprising the step consisting of i) implementing the method of the present disclosure for assessing the ability of CTL to provide a cytotoxic response and the ability of the tumor cells to resist to the cytotoxic response; b) implementing an algorithm on data comprising the lytic potentials determined at step i) so as to obtain an algorithm output; c) determining the probability that the patient will achieve a response to the treatment.

The algorithm of the present disclosure can be performed by one or more programmable processors executing one or more computer programs to perform functions by operating on input data and generating output. The algorithm can also be performed by, and apparatus can also be implemented as, special purpose logic circuitry, e.g., an FPGA (field programmable gate array) or an ASIC (application-specific integrated circuit). Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for performing instructions and one or more memory devices for storing instructions and data. Generally, a computer will also include, or be operatively coupled to receive data from or transfer data to, or both, one or more mass storage devices for storing data, e.g., magnetic, magneto-optical disks, or optical disks. However, a computer need not have such devices. Moreover, a computer can be embedded in another device. Computer-readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto-optical disks; and CD-ROM and DVD-ROM disks. The processor and the memory can be supplemented by, or incorporated in, special purpose logic circuitry. To provide for interaction with a user, the method of the present disclosure can be implemented on a computer having a display device, e.g., in non-limiting examples, a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. Accordingly, in particular, the algorithm can be implemented in a computing system that includes a back-end component, e.g., as a data server, or that includes a middleware component, e.g., an application server, or that includes a front-end component, e.g., a client computer having a graphical user interface or a Web browser through which a user can interact with an implementation of the disclosure, or any combination of one or more such back-end, middleware, or front-end components. The components of the system can be interconnected by any form or medium of digital data communication, e.g., a communication network. Examples of communication networks include a local area network ("LAN") and a wide area network ("WAN"), e.g., the Internet. The computing system can include clients and servers. A client and server are generally remote from each other and typically interact through a communication network. The relationship of client and server arises by virtue of computer programs running on the respective computers and having a client-server relationship to each other.

Other aspects of the present disclosure include kits for carrying out the method of the present disclosure. A kit may include materials useful in preparing immune effector cells and target cells, the fluorescent dye, a device such as a 96-well plate in which the target and effector cells may be mixed and incubated, materials required detecting the fluorescence intensity. Different combinations of such materials may be organized as a kit in order to aid the skilled artisan in carrying out the method of the present disclosure. In particular, the kit of the present disclosure further comprises a microprocessor to implement the algorithm as described above and a visual display and/or audible signal that indicates the probability determined by the microprocessor.

The disclosure will be further illustrated by the following figures and examples.

### FIGURES:

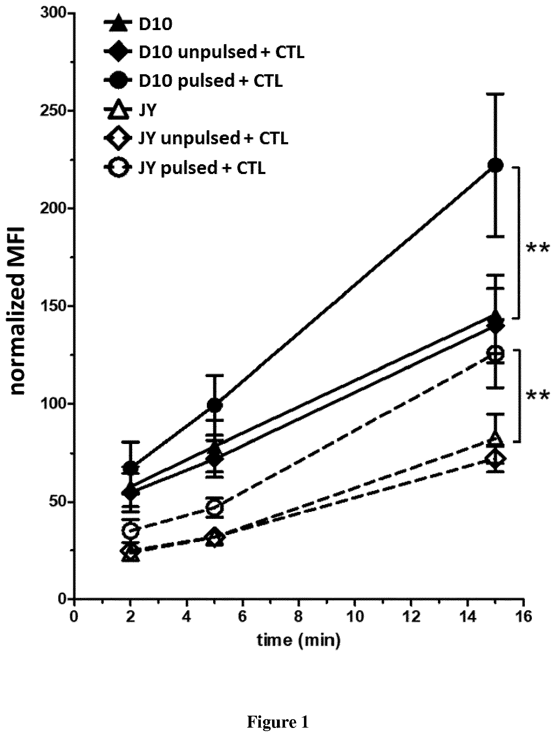

**Figure 1****: FM1-43 internalization is enhanced after CTL attack in resistant and sensitive target cells.** Time kinetics of FM1-43 fluorescence intensity in D10 or JY cells unpulsed or pulsed with 10µM antigenic peptide. Analysis was performed on target cells either alone or following conjugation with CTL during 2, 5 or 15min. Normalized MFI corresponds to: geometric mean fluorescence intensity of the sample - geometric mean of unstained target cells. Results are expressed as mean ± SEM of 4 independent experiments performed in duplicate. Unpaired Mann Whitney test using GraphPad Prism software was used to determine the statistical significance after 15min of conjugation. **P<0,01

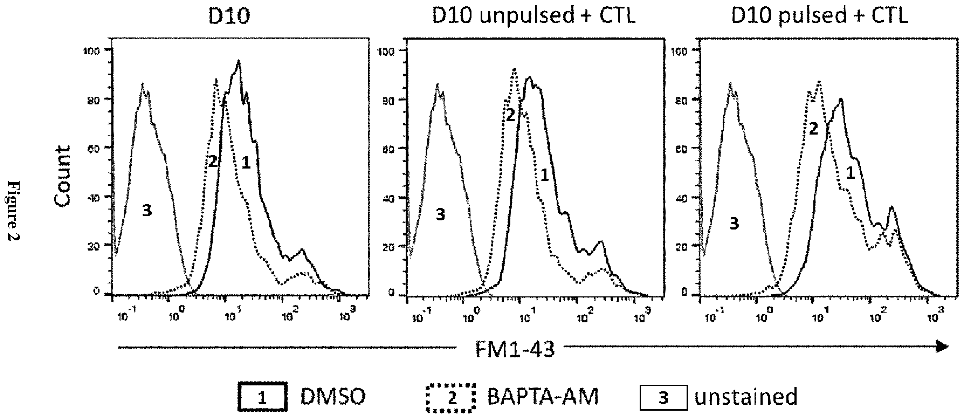

**Figure 2****: BAPTA-AM pretreatment decreases FM1-43 internalization in melanoma cells.** FACS plot analysis of FM1-43 fluorescence intensity in melanoma cells (D10) pretreated or not with 50µM BAPTA-AM either alone or following 5 min conjugation with CTL. Presented are typical results from one experiment out of three.

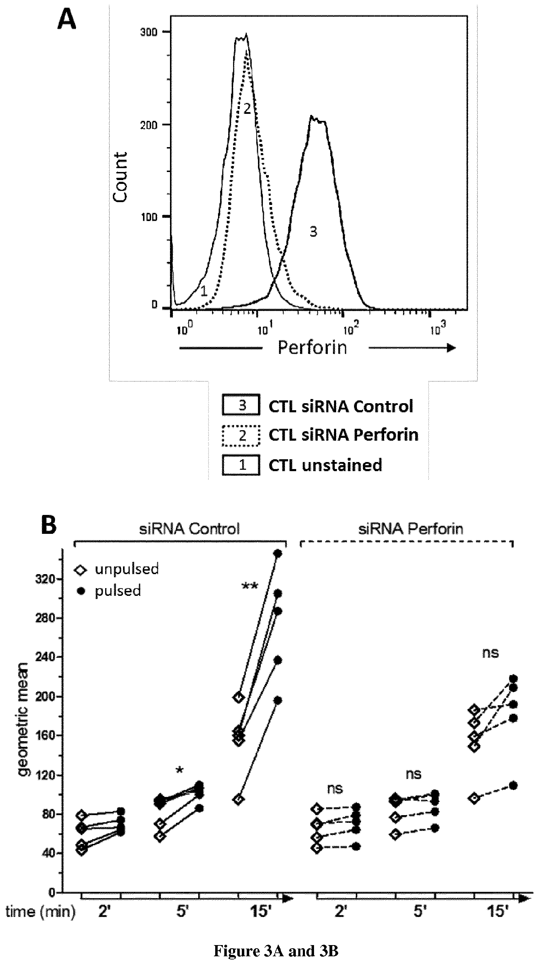

**Figure 3****: Perforin silencing significantly decreases membrane response in melanoma cells.** (a) FACS plot analysis of perforin fluorescence intensity in CTL previously electroporated with siRNA targeting perforin or control siRNA. (b) Time kinetics of FM1-43 fluorescence intensity in D10 cells either unpulsed (empty symbols) or pulsed (filled symbols) following conjugation during 2, 5 and 15min with CTL previously electroporated with siRNA targeting perforin or control siRNA. Data are from five independant experiments (three independant electroporation). Unpaired Mann Whitney test using GraphPad Prism software was used to determine the statistical significance. ns *P*>0.05 **P*<0.05 ***P*<0.01

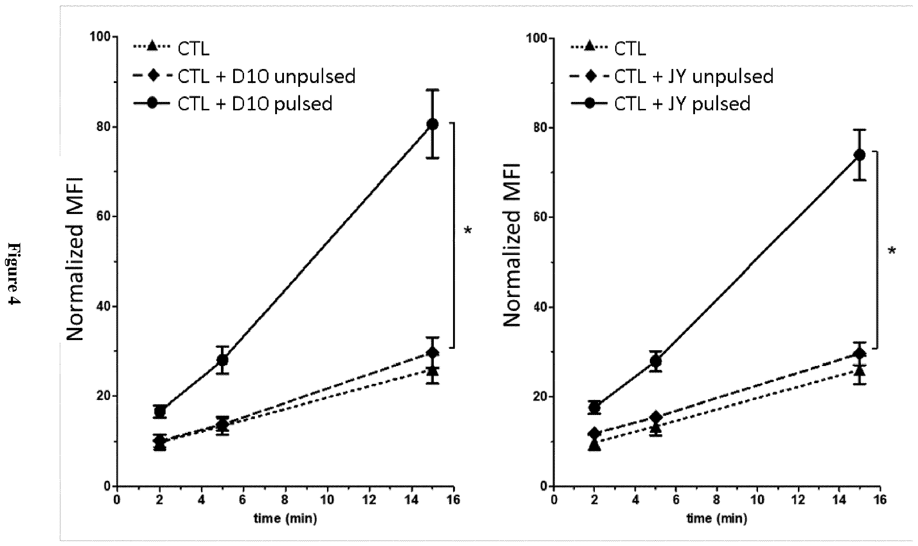

**Figure 4****: FM1-43 internalization is equally enhanced in CTL after activation by resistant or sensitive target cells.** (a,b) Time kinetics of FM1-43 fluorescence intensity in CTL either alone or following conjugation with D10 (a) or JY cells (b) during 2, 5 or 15min. Normalized MFI corresponds to: geometric mean fluorescence intensity of the sample - geometric mean of unstained CTL. Results are expressed as mean ± SEM of 4 independent experiments performed in duplicate, same experiments as in Figure 1. Unpaired Mann Whitney test using GraphPad Prism software was used to determine the statistical significance after 15min of conjugation. **P*<0,05

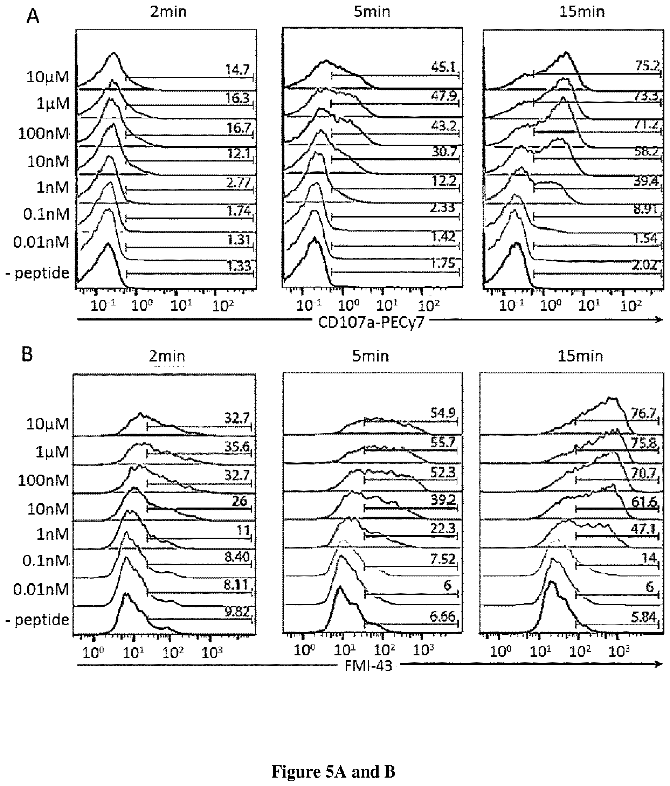

**Figure 5****: FMI-43 uptake in CTL as compared to CD107a exposure to measure CTL activation following antigenic stimulation.** Target cells were either unpulsed or pulsed with different concentrations of antigenic peptide and conjugated with cognate CTL for 2, 5 or 15min. (**A**) After conjugation CD107a exposure was quantified in CTL by flow cytometry. Indicated numbers are percentages of CD107a⁺ cells. (**B**) During conjugation, 10µg/ml of FM1-43 was added into the medium. FM1-43 fluorescence intensities are shown in the CTL side of the lytic synapse. Numbers indicate percentages of cells with an increase in FM1-43 uptake compared to the unpulsed condition. These results are typical of two independent experiments.

### EXAMPLE:

### Methods:

### Cells

An HLA-A2- restricted human CD8⁺ CTL clone specific for the NLVPMVATV peptide (SEQ ID NO:1) of the cytomegalovirus protein pp65 was used. HLA-A2⁺ EBV-transformed human B cells (JY cells) and melanoma cell line (D10) were used as target cells. T cell clones and EBV-B cell lines were generated and maintained as described (Khazen et al., 2016). D10 cells were kindly provided by Dr G. Spagnoli, Basel, Switzerland).

### Perforin silencing

Using a square wave a Gene PulserXcell electroporation system (BioRad), 1.10⁶ CTL (in 100µl OptiMEM medium, Gibco) were electroporated (300V, 2msec) with siRNA (300pmol) targeting perforin or control siRNA. CTL were transferred into warm complete RPMI/HS culture medium, incubated at 37°C/5%CO₂ and used 48h or 72h after electroporation. Silencing efficacy was checked at the RNA level by RT-qPCR experiments (not shown) and at the protein level following intracellular staining by flow cytometry.

### Perforin staining by flow cytometry

CTL previously siRNA transfected were fixed with 3% paraformaldehyde, permeabilized with 0.1% saponin (in PBS/3%BSA/HEPES), and stained with an anti-human perforin mAb (10µg/ml, clone δG9, BD Biosciences). The primary antibody was followed by goat anti-mouse isotype specific Ab labeled with Alexa 488. Samples were acquired using a FACS MACSQuant 10 (MiltenyiBiotec).

### Membrane turnover assay

Loading of target cells: Target cells were left unpulsed or pulsed with 10µM antigenic peptide during 2h at 37°C/5% CO₂. Cells were washed three times, re-suspended at 15×10³to 30×10³cells in 25µl RPMI/5% FCS/HEPES and transferred to a 96-well U-bottom plate.

In some experiments, D10 cells were pretreated (2h) or not with 50µM BAPTA-AM (ThermoFisher Scientific).

*CTL staining:* CTL were stained with 10µMCellTrace Violet (CTV, ThermoFisher Scientific) for 30min at 37°C/5% CO₂, washed three times and re-suspended at 30×10³ to 60×10³cells per 25µl RPMI/5% FCS/HEPES.

*Conjugation of CTL and target cells:* 25 µl of CTL (previously stained with CTV) were added in wells containing pulsed or unpulsed target cells at 2:1 CTL/target cell ratio. Target cells were previously added to three 96-U-bottom plates (one plate per time point: 2, 5 or 15 minutes). A pre-diluted solution of FM1-43 (ThermoFisher Scientific) was prepared at 20µg/ml. 50µl of this solution were added to wells containing in 50µl the cells of interest to have a final concentration of 10µg/ml. The FM1-43 solution was added to wells at the same time of CTL. Cells were pelleted during 1 minute at 455g and incubated at 37°C/5% CO₂ for 2, 5 or 15 minutes.

At the end of the incubation time, 100µl cold FACS buffer (PBS 1%FBS 1%HS 0.1% azide) supplemented with 0.5mM EDTA were added to each well on ice. Cells were pelleted for 2min at 455g at4°C and washed with 200µl FACS buffer/ 0.5mM EDTA.

Viability staining: After washing, Fixable viability dye eFluor780 (ThermoFisher Scientific, 1000X dilution according to manufacturer instructions) was added to each well in 50µlFACS buffer. Cells were kept on ice during 20 minutes and washed twice in FACS buffer. Samples were acquired using a FACS MACSQuant 10 (MiltenyiBiotec).

### CTL CD107a exposure

JY cells were left unpulsed or pulsed with 0.01nM to 10µM of antigenic peptide during 2h at 37°C/5% CO₂, washed three times and subsequently transferred to a 96-well U-bottom plate at 20 × 10³ cells per 50µl RPMI 5% FCS/HEPES. CTL were previously stained with 0.1µM CMFDA for 20min at 37°C/5% CO₂, washed and added to the target cells at two CTL versus one target cell ratio in 50µl RPMI 5% FCS/HEPES. Cells were pelleted for 1min, 455g and incubated at 37°C/5% CO₂ for 2min, 5min or 15min. At the end of each incubation time, CTL/target cell co-cultures were resuspended and washed in ice-cold PBS containing 2mM EDTA. Cells were stained with fixable viability dye eFluor 450 (eBiosciences) and with anti-human CD107a PE-Cy7 (10µg/ml; BD Biosciences) on ice at 4°C for 30min in FACS buffer. Samples were acquired using MACS Quant Analyzer VYB (Miltenyi). Results were analyzed using the FlowJo 10 software.

### Results:

To directly detect the earliest steps of tumor cell resistance to CTL attack at the lytic synapse we developed a method to monitor CTL/tumor cells interaction in the presence of FM1-43, a fluorescent lipophilic dye that rapidly intercalates into lipid bilayer during the membrane turnover that accompanies plasma membrane reparation. This assay allows us to measure reparative membrane turnover of tumor cells under CTL attack by FACS analysis at the whole tumor cell population level. We initially applied this approach to compare the response of melanoma cell (D10 cells) to CTL attack as compared to conventional target cells that are sensitive to CTL mediated cytotoxicity (EBV-transformed B cells, JY cells). As shown in **Figure 1****,** we observed that, in the absence of CTL and in conditions in which target cells were not loaded with the specific antigenic peptide, FM1-43 intercalated into the D10 membrane with a faster rate when compare to JY cells, indicating that melanoma cells exhibit a more active basal membrane recycling than conventional target cells. Upon CTL attack, the rate of FM1-43 uptake increased in both target cells, indicating that CTL attack triggers a membrane reparation activity that enhances the basal FM1-43 uptake.

Together, the above results show that our method can be used to monitor the basal membrane turnover and the kinetics and extend of target cell response to CTL attack by FACS analysis.

They also show that resistant target cells such as melanoma cells have a faster membrane turnover, both in basal conditions and upon CTL attack, suggesting that this mechanism might contribute to target cell resistance to perforin-mediated cytotoxicity.

We next investigated whether FM1-43 membrane uptake in basal conditions and following CTL attack wasCa²⁺ dependent. To this end, D10 cells, previously loaded with the antigenic peptide, were pretreated or not with 50µm BAPTA-AM (a Ca²⁺ chelator) and conjugated with CTL. We observed a decrease of FM1-43 uptake in BAPTA-AM pretreated cells when compared to untreated cells (**Figure 2**). These results support the notion that the observed membrane reparative turnover is part of an ancestral membrane reparation process based on extracellular Ca²⁺ entry and membrane sealing via the activation of lysosome exocytosis and membrane turnover (Cheng et al., 2015).

We finally investigated whether the observed induction of membrane reparative turnover in target cells would be a direct consequence of perforin-mediated pore formation in their plasma membrane. To address this point, perforin expression was silenced in CTL using a siRNA approach (**Figure 3** **A**). Under these conditions, melanoma cell reparative response was reduced (**Figure 3** **B**). Together, these results indicate that the triggering of membrane reparation is directly related to the CTL lytic potential.

An interesting aspect of our assay is that, by gating on CTL (see materials and methods section), we can in the same sample verify the level of activation of these cells following conjugation with target cells. Indeed, upon TCR engagement, CTL undergo the activation of their endocytic/exocytic pathway resulting in FM1-43 uptake. FM1-43 uptake in CTL can be used to verify if a given target cell population has efficiently triggered lytic granule secretion in CTL. For instance, in **Figure 4** it is shown that, although melanoma cells undergo exacerbated membrane reparation when compare to JY cells, both cells similarly trigger lytic granule secretion in CTL.

We propose to use FM1-43 uptake in CTL/target cell conjugates to detect not only target cell defence response at the lytic synapse, but also the rapid activation of CTL. Several methods have been described to detect CTL activation by FACS analysis. Among those methods the golden standard is the up-regulation of the lysosomal marker CD107a on the surface of CTL (Bertrand et al, 2013). The exposure of this marker on the cell surface indicates that the CTL has secreted its lytic granule and is somehow considered the "smoking gun" of the CTL.

We compared FM1-43 to CD107a exposure in a cohort of CTL stimulated by conventional target cells (JY EBV-transformed B cells) pulsed with increasing concentration of the antigenic peptide. As shown in **Figure 5A-B,** this analysis revealed that FM1-43 uptake is more rapid (is detected earlier) and more sensitive (is detected at lower antigenic concentrations) when compared to the golden standard CD107a exposure.

It is also important to note that, being based on the addition of a fluorescent probe to culture medium the FM1-43 uptake method is less expensive of the measurement of CD107a exposure that is based on the use of fluorochrome-labelled antibodies and can be used on cellular suspensions of cells derived from primary tumors without any manipulation of the cells. This is obviously not possible when staining for CD107a since cells need to be washed and kept at 4°C for staining. Variant of staining for CD107a exist in which anti-CD107a fluorochrome-labelled antibodies are added to culture medium in a sort of live cell loading procedure, but these methods normally require hours of cell-cell interaction to provide detectable results (Betts et al., 2003). They are therefore largely less efficient of the FM1-43 uptake method.

In conclusion the FM1-43 uptake technique has the qualities requested to become the golden standard to measure human CTL degranulation in health and disease. Indeed the measurement of FM1-43 uptake is more efficient, rapid and less expensive than measurement of CD107a exposure to assess CTL activation.

### REFERENCES:

Throughout this application, various references describe the state of the art to which this disclosure pertains.
Andrews, N.W., Almeida, P.E., and Corrotte, M. (2014). Damage control: cellular mechanisms of plasma membrane repair. Trends Cell Biol. 24, 734-742.
Baran, K., Dunstone, M., Chia, J., Ciccone, A., Browne, K.A., Clarke, C.J.P., Lukoyanova, N., Saibil, H., Whisstock, J.C., Voskoboinik, I., et al. (2009). The Molecular Basis for Perforin Oligomerization and Transmembrane Pore Assembly. Immunity 30, 684-695.
Betts, M.R., Brenchley, J.M., Price, D.A., De Rosa, S.C., Douek, D.C., Roederer, M., Koup, R.A. (2003). Sensitive and viable identification of antigen-specific CD8+ T cells by a flow cytometric assay for degranulation. Journal of Immunological Methods. 281, 65-78.
Bertrand, F., Muller, S., Roh, K.-H., Laurent, C., Dupre, L., and Valitutti, S. (2013). An initial and rapid step of lytic granule secretion precedes microtubule organizing center polarization at the cytotoxic T lymphocyte/target cell synapse. Proc. Natl. Acad. Sci. 110, 6073-6078.
Caramalho, Í., Faroudi, M., Padovan, E., Müller, S., and Valitutti, S. (2009). Visualizing CTL/melanoma cell interactions: multiple hits must be delivered for tumour cell annihilation. J. Cell. Mol. Med. 13, 3834-3846.
Chen, D.S., and Mellman, I. (2017). Elements of cancer immunity and the cancer-immune set point. Nature 541, 321-330.
Cheng, X., Zhang, X., Yu, L., and Xu, H. (2015). Calcium signaling in membrane repair. Semin. Cell Dev. Biol. 45, 24-31.
Faroudi, M., Zaru, R., Paulet, P., Muller, S., and Valitutti, S. (2003). Cutting Edge: T Lymphocyte Activation by Repeated Immunological Synapse Formation and Intermittent Signaling. J. Immunol. 171, 1128-1132.
Horn, A., and Jaiswal, J.K. (2018). Cellular mechanisms and signals that coordinate plasma membrane repair. Cell. Mol. Life Sci. 75, 3751-3770.
Khazen, R., Müller, S., Gaudenzio, N., Espinosa, E., Puissegur, M.-P., and Valitutti, S. (2016). Melanoma cell lysosome secretory burst neutralizes the CTL-mediated cytotoxicity at the lytic synapse. Nat. Commun. 7, 10823.
Law, R.H.P., Lukoyanova, N., Voskoboinik, I., Caradoc-Davies, T.T., Baran, K., Dunstone, M.A., D'Angelo, M.E., Orlova, E.V., Coulibaly, F., Verschoor, S., et al. (2010). The structural basis for membrane binding and pore formation by lymphocyte perforin. Nature 468, 447-451.
Lopez, J.A., Susanto, O., Jenkins, M.R., Lukoyanova, N., Sutton, V.R., Law, R.H., Johnston, A., Bird, C.H., Bird, P.I., and Whisstock, J.C. (2013). Perforin forms transient pores on the target cell plasma membrane to facilitate rapid access of granzymes during killer cell attack. Blood 121, 2659-2668.
de Saint Basile, G., Ménasché, G., and Fischer, A. (2010). Molecular mechanisms of biogenesis and exocytosis of cytotoxic granules. Nat. Rev. Immunol. 10, 568-579.
Schumacher, T.N., Kesmir, C., and van Buuren, M.M. (2015). Biomarkers in Cancer Immunotherapy. Cancer Cell 27, 12-14.
Stinchcombe, J.C., Bossi, G., Booth, S., and Griffiths, G.M. (2001). The immunological synapse of CTL contains a secretory domain and membrane bridges. Immunity 15, 751-761.
Thiery, J., Keefe, D., Boulant, S., Boucrot, E., Walch, M., Martinvalet, D., Goping, I.S., Bleackley, R.C., Kirchhausen, T., and Lieberman, J. (2011). Perforin pores in the endosomal membrane trigger the release of endocytosed granzyme B into the cytosol of target cells. Nat. Immunol. 12, 770-777.

## Claims

1. An in vitro method of assaying the lytic potential of a population of immune effector cells comprising the steps consisting of i) contacting the population of immune effector cells with a population of target cells for a sufficient period of time and under conditions suitable for allowing the population of immune effector cells to induce a cytotoxic response, ii) measuring the reparative membrane turnover level in the population of target cells by measuring the uptake of a fluorescent lipophilic dye by the population of target cells, wherein said level correlates with the lytic potential of the population of immune effector cells and wherein the fluorescent lipophilic dye is a styryl dye.

2. The method of claim 1 wherein the immune effector cells are selected from the group consisting of cytotoxic T lymphocytes (CTLs), natural killer (NK) cells, and natural killer T (NKT) cells.

3. The method of claim 1 or 2 wherein the immune effector cells are tumor infiltrating cytotoxic T lymphocytes.

4. The method of claim 1 wherein the target cells are tumor cells.

5. The method of claim 1 wherein the target cells are previously pulsed with at least one antigen.

6. The method of claim 5 wherein the target cells are pulsed with a peptide corresponding to the amino acid sequence of an infectious agent or a tumor antigen.

7. The method of claim 1 wherein the immune effectors cells and/or the target cells are isolated from a biological sample obtained from a patient.

8. The method of claim 7 wherein the biological sample is a tissue sample such as a tumor tissue sample, or a body fluid sample such as a blood sample.

9. The method of claim 1 which further comprises the step consisting of measuring membrane turnover level in the population of immune effector cells.

10. The method of claim 1 which further comprises the step of measuring the uptake of the fluorescent lipophilic dye by the population of immune effector cells.

11. The method of claim 1 wherein the styryl dye is selected from the group consisting of FM1-43, FM4-64, FM14-68, FM2-10, FM4-84, FM1-84, FM14-27, FM14-29, FM3-25, FM3-14, FM5-55, RH414, FM6-55, FM10-75, FM1-81, FM9-49, FM4-95, FM4-59, and FM9-40.

12. The method of claim 1 wherein the fluorescent dye uptake is measured using devices that measure cell fluorescence, such as a Fluorescence Activated Cell Sorter (FACS) machine.

13. The method of claim 1 which comprises the step consisting of comparing the reparative membrane turnover level to a reference value.

14. Use of the method of claim 1 in a diagnostic assay.

15. An in vitro Or method of screening a test compound for the ability to modulate the lytic potential of a population of immune effector cells comprising the steps consisting of i) contacting the population of immune effector cells with a population of target cells for a sufficient period of time and under conditions suitable for allowing the population of immune effector cells to induce a cytotoxic response in presence of the test compound, ii) measuring the reparative membrane turnover level in the population of target cells by measuring the uptake of a fluorescent lipophilic dye by the population of target cells, iii) comparing the reparative membrane turnover level determined at step ii) with the reparative membrane turnover level measured in absence of the test compound and iv) selecting the test compound wherein a difference is detected between the reparative membrane turnover level determined at step ii) and the reparative membrane turnover level measured in absence of the test compound, wherein the fluorescent lipophilic dye is a styryl dye.

16. The method of claim 15 wherein the styryl dye is selected from the group consisting of FM1-43, FM4-64, FM14-68, FM2-10, FM4-84, FM1-84, FM14-27, FM14-29, FM3-25, FM3-14, FM5-55, RH414, FM6-55, FM10-75, FM1-81, FM9-49, FM4-95, FM4-59, and FM9-40.

17. The method of claim 15 wherein the test compound has been previously selected to modulate the activity or expression of an immune checkpoint protein.

18. Use of the method of claim 1 for detecting T cell exhaustion in a patient.

19. A method of determining whether a patient suffering from cancer will achieve a response with an immune checkpoint inhibitor, comprising the steps consisting of i) isolating a population of immune effector cells from a biological sample obtained from the patient, ii) contacting the population of immune effector cells with a population of target cells for a sufficient period of time and under conditions suitable for allowing the population of immune effector cells to induce a cytotoxic response in presence of the immune checkpoint inhibitor, iii) measuring the reparative membrane turnover level in the population of target cells by measuring the uptake of a fluorescent lipophilic dye by the population of target cells, iv) comparing the reparative membrane turnover level determined at step iii) with the reparative membrane turnover level measured in absence of the immune checkpoint inhibitor and v) concluding that the patient will achieve a response with the immune checkpoint inhibitor when the reparative membrane turnover level determined at step iii) is higher than the reparative membrane turnover level measured in absence of the immune checkpoint inhibitor, wherein the fluorescent lipophilic dye is a styryl dye.

20. The method of claim 19 wherein the styryl dye is selected from the group consisting of FM1-43, FM4-64, FM14-68, FM2-10, FM4-84, FM1-84, FM14-27, FM14-29, FM3-25, FM3-14, FM5-55, RH414, FM6-55, FM10-75, FM1-81, FM9-49, FM4-95, FM4-59, and FM9-40.

21. A method of determining whether a patient suffering from a cancer will achieve a response with a treatment comprising the step consisting of i) implementing the method of claim 1 for assessing the ability of CTL to provide a cytotoxic response and the ability of the tumor cells to resist to the cytotoxic response; b) implementing an algorithm on data comprising the lytic potentials determined at step i) so as to obtain an algorithm output; c) determining the probability that the patient will achieve a response to the treatment.

## Patentansprüche

1. In-vitro-Verfahren zur Untersuchung des lytischen Potenzials einer Population von Immuneffektorzellen, umfassend die Schritte bestehend aus i) Inkontaktbringen der Population von Immuneffektorzellen mit einer Population von Zielzellen über einen ausreichenden Zeitraum und unter Bedingungen, die geeignet sind, der Population von Immuneffektorzellen zu erlauben, eine zytotoxische Reaktion zu induzieren, ii) Messen des reparativen Membranumsatzniveaus in der Population von Zielzellen durch Messen der Aufnahme eines fluoreszierenden lipophilen Farbstoffs durch die Population von Zielzellen, wobei das Niveau mit dem lytischen Potential der Population von Immuneffektorzellen korreliert und wobei der fluoreszierende lipophile Farbstoff ein Styrylfarbstoff ist.

2. Verfahren nach Anspruch 1, wobei die Immuneffektorzellen aus der Gruppe bestehend aus zytotoxischen T-Lymphozyten (CTLs), natürlichen Killerzellen (NK) und natürlichen Killer-T-Zellen (NKT) ausgewählt sind.

3. Verfahren nach Anspruch 1 oder 2, wobei die Immuneffektorzellen tumorinfiltrierende zytotoxische T-Lymphozyten sind.

4. Verfahren nach Anspruch 1, wobei die Zielzellen Tumorzellen sind.

5. Verfahren nach Anspruch 1, wobei die Zielzellen zuvor mit mindestens einem Antigen gepulst werden.

6. Verfahren nach Anspruch 5, wobei die Zielzellen mit einem Peptid gepulst werden, das der Aminosäuresequenz eines Infektionserregers oder eines Tumorantigens entspricht.

7. Verfahren nach Anspruch 1, wobei die Immuneffektoren und/oder die Zielzellen aus einer biologischen Probe isoliert werden, die einem Patienten entnommen wird.

8. Verfahren nach Anspruch 7, wobei die biologische Probe eine Gewebeprobe, wie eine Tumorgewebeprobe, oder eine Körperflüssigkeitsprobe, wie eine Blutprobe, ist.

9. Verfahren nach Anspruch 1, das weiter den Schritt umfasst, der darin besteht, das Membranumsatzniveau in der Population von Immuneffektorzellen zu messen.

10. Verfahren nach Anspruch 1, das weiter den Schritt des Messens der Aufnahme des fluoreszierenden lipophilen Farbstoffs durch die Population von Immuneffektorzellen umfasst.

11. Verfahren nach Anspruch 1, wobei der Styrylfarbstoff ausgewählt ist aus der Gruppe bestehend aus FM1-43, FM4-64, FM14-68, FM2-10, FM4-84, FM1-84, FM14-27, FM14-29, FM3-25, FM3-14, FM5-55, RH414, FM6-55, FM10-75, FM1-81, FM9-49, FM4-95, FM4-59 und FM9-40.

12. Verfahren nach Anspruch 1, wobei die Aufnahme des Fluoreszenzfarbstoffs mit Hilfe von Vorrichtungen gemessen wird, die die Zellfluoreszenz messen, wie einem Fluoreszenz-aktivierten Zellsortiergerät (FACS).

13. Verfahren nach Anspruch 1, das den Schritt umfasst, der darin besteht, das reparative Membranumsatzniveau mit einem Referenzwert zu vergleichen.

14. Verwendung des Verfahrens nach Anspruch 1 bei einem diagnostischen Test.

15. In-vitro-Verfahren zum Screenen einer Testverbindung auf die Fähigkeit, das lytische Potenzial einer Population von Immuneffektorzellen zu modulieren, umfassend die Schritte bestehend aus i) Inkontaktbringen der Population von Immuneffektorzellen mit einer Population von Zielzellen über einen ausreichenden Zeitraum und unter Bedingungen, die geeignet sind, der Population von Immuneffektorzellen zu erlauben, eine zytotoxische Reaktion in Gegenwart der Testverbindung zu induzieren, ii) Messen des reparativen Membranumsatzniveaus in der Population von Zielzellen durch Messen der Aufnahme eines fluoreszierenden lipophilen Farbstoffs durch die Population von Zielzellen, iii) Vergleichen des in Schritt ii) bestimmten reparativen Membranumsatzniveaus mit dem in Abwesenheit der Testverbindung gemessenen reparativen Membranumsatzniveau und iv) Auswählen der Testverbindung, wobei ein Unterschied zwischen dem in Schritt ii) bestimmten reparativen Membranumsatzniveau und dem in Abwesenheit der Testverbindung gemessenen reparativen Membranumsatzniveau nachgewiesen wird, wobei der fluoreszierende lipophile Farbstoff ein Styrylfarbstoff ist.

16. Verfahren nach Anspruch 15, wobei der Styrylfarbstoff ausgewählt ist aus der Gruppe bestehend aus FM1-43, FM4-64, FM14-68, FM2-10, FM4-84, FM1-84, FM14-27, FM14-29, FM3-25, FM3-14, FM5-55, RH414, FM6-55, FM10-75, FM1-81, FM9-49, FM4-95, FM4-59 und FM9-40.

17. Verfahren nach Anspruch 15, wobei die Testverbindung zuvor ausgewählt wurde, um die Aktivität oder Expression eines Immun-Checkpoint-Proteins zu modulieren.

18. Verwendung des Verfahrens nach Anspruch 1 zum Nachweis einer T-Zell-Erschöpfung bei einem Patienten.

19. Verfahren zur Untersuchung, ob ein Patient, der an Krebs leidet, eine Reaktion mit einem Immun-Checkpoint-Inhibitor erzielen wird, umfassend die Schritte bestehend aus i) Isolieren einer Population von Immuneffektorzellen aus einer biologischen Probe, die dem Patienten entnommen wurde, ii) Inkontaktbringen der Population von Immuneffektorzellen mit einer Population von Zielzellen für einen ausreichenden Zeitraum und unter Bedingungen, die geeignet sind, der Population von Immuneffektorzellen zu erlauben, eine zytotoxische Reaktion in Gegenwart des Immun-Checkpoint-Inhibitors zu induzieren, iii) Messen des reparativen Membranumsatzniveaus in der Population von Zielzellen durch Messen der Aufnahme eines fluoreszierenden lipophilen Farbstoffs durch die Population von Zielzellen, iv) Vergleichen des in Schritt iii) bestimmten reparativen Membranumsatzniveaus mit dem in Abwesenheit des Immun-Checkpoint-Inhibitors gemessenen reparativen Membranumsatzniveau und v) Schließen, dass der Patient eine Reaktion mit dem Immun-Checkpoint-Inhibitor erzielen wird, wenn das in Schritt iii) bestimmte reparative Membranumsatzniveau höher ist als das in Abwesenheit des Immun-Checkpoint-Inhibitors gemessene reparativen Membranumsatzniveau, wobei der fluoreszierende lipophile Farbstoff ein Styryl-Farbstoff ist.

20. Verfahren nach Anspruch 19, wobei der Styrylfarbstoff ausgewählt ist aus der Gruppe bestehend aus FM1-43, FM4-64, FM14-68, FM2-10, FM4-84, FM1-84, FM14-27, FM14-29, FM3-25, FM3-14, FM5-55, RH414, FM6-55, FM10-75, FM1-81, FM9-49, FM4-95, FM4-59 und FM9-40.

21. Verfahren zum Bestimmen, ob ein Patient, der an einer Krebserkrankung leidet, auf eine Behandlung ansprechen wird, umfassend den Schritt bestehend aus i) Durchführen des Verfahrens nach Anspruch 1 zum Bewerten der Fähigkeit von CTL, eine zytotoxische Reaktion bereitzustellen, und der Fähigkeit der Tumorzellen, der zytotoxischen Reaktion zu widerstehen; b) Durchführen eines Algorithmus an Daten, die die in Schritt i) bestimmten lytischen Potenziale umfassen, um eine Algorithmusausgabe zu erhalten; c) Bestimmen der Wahrscheinlichkeit, dass der Patient auf die Behandlung ansprechen wird.

## Revendications

1. Procédé in vitro de dosage du potentiel lytique d'une population de cellules effectrices immunitaires comprenant les étapes consistant à i) mettre en contact la population de cellules effectrices immunitaires avec une population de cellules cibles pendant une période de temps suffisante et dans des conditions appropriées pour permettre à la population de cellules effectrices immunitaires d'induire une réponse cytotoxique, ii) mesurer le niveau de renouvellement de membrane réparatrice dans la population de cellules cibles en mesurant l'absorption d'un colorant lipophile fluorescent par la population de cellules cibles, dans lequel ledit niveau est en corrélation avec le potentiel lytique de la population de cellules effectrices immunitaires et dans lequel le colorant lipophile fluorescent est un colorant styryle.

2. Procédé selon la revendication 1 dans lequel les cellules effectrices immunitaires sont choisies dans le groupe consistant en lymphocytes T cytotoxiques (CTL), cellules tueuses naturelles (NK) et cellules T tueuses naturelles (NKT).

3. Procédé selon la revendication 1 ou 2 dans lequel les cellules effectrices immunitaires sont des lymphocytes T cytotoxiques infiltrant une tumeur.

4. Procédé selon la revendication 1 dans lequel les cellules cibles sont des cellules tumorales.

5. Procédé selon la revendication 1 dans lequel les cellules cibles sont préalablement pulsées avec au moins un antigène.

6. Procédé selon la revendication 5 dans lequel les cellules cibles sont pulsées avec un peptide correspondant à la séquence d'acides aminés d'un agent infectieux ou d'un antigène tumoral.

7. Procédé selon la revendication 1 dans lequel les cellules effectrices immunitaires et/ou les cellules cibles sont isolées d'un échantillon biologique obtenu auprès d'un patient.

8. Procédé selon la revendication 7 dans lequel l'échantillon biologique est un échantillon de tissu tel qu'un échantillon de tissu tumoral, ou un échantillon de fluide corporel tel qu'un échantillon de sang.

9. Procédé selon la revendication 1 qui comprend en outre l'étape consistant à mesurer le niveau de renouvellement de membrane dans la population de cellules effectrices immunitaires.

10. Procédé selon la revendication 1 qui comprend en outre l'étape consistant à mesurer l'absorption du colorant lipophile fluorescent par la population de cellules effectrices immunitaires.

11. Procédé selon la revendication 1 dans lequel le colorant styryle est choisi dans le groupe consistant en FM1-43, FM4-64, FM14-68, FM2-10, FM4-84, FM1-84, FM14-27, FM14-29, FM3-25, FM3-14, FM5-55, RH414, FM6-55, FM10-75, FM1-81, FM9-49, FM4-95, FM4-59 et FM9-40.

12. Procédé selon la revendication 1 dans lequel l'absorption du colorant fluorescent est mesurée à l'aide de dispositifs qui mesurent la fluorescence cellulaire, comme un trieur de cellules à fluorescence (FACS).

13. Procédé selon la revendication 1 qui comprend l'étape consistant à comparer le niveau de renouvellement de membrane réparatrice à une valeur de référence.

14. Utilisation du procédé selon la revendication 1 dans un dosage de diagnostic.

15. Procédé in vitro de criblage d'un composé de test pour sa capacité à moduler le potentiel lytique d'une population de cellules effectrices immunitaires comprenant les étapes consistant à i) mettre en contact la population de cellules effectrices immunitaires avec une population de cellules cibles pendant une période de temps suffisante et dans des conditions appropriées pour permettre à la population de cellules effectrices immunitaires d'induire une réponse cytotoxique en présence du composé de test, ii) mesurer le niveau de renouvellement de membrane réparatrice dans la population de cellules cibles en mesurant l'absorption d'un colorant lipophile fluorescent par la population de cellules cibles, iii) comparer le niveau de renouvellement de membrane réparatrice déterminé à l'étape ii) avec le niveau de renouvellement de membrane réparatrice mesuré en l'absence du composé de test, et iv) sélectionner le composé de test dans lequel une différence est détectée entre le niveau de renouvellement de membrane réparatrice déterminé à étape ii) et le niveau de renouvellement de membrane réparatrice mesuré en l'absence du composé de test, dans lequel le colorant lipophile fluorescent étant un colorant styryle.

16. Procédé selon la revendication 15 dans lequel le colorant styryle est choisi dans le groupe consistant en FM1-43, FM4-64, FM14-68, FM2-10, FM4-84, FM1-84, FM14-27, FM14-29, FM3-25, FM3-14, FM5-55, RH414, FM6-55, FM10-75, FM1-81, FM9-49, FM4-95, FM4-59 et FM9-40.

17. Procédé selon la revendication 15 dans lequel le composé de test a été préalablement sélectionné pour moduler l'activité ou l'expression d'une protéine de point de contrôle immunitaire.

18. Utilisation du procédé selon la revendication 1 pour détecter l'épuisement des lymphocytes T chez un patient.

19. Procédé permettant de déterminer si un patient souffrant d'un cancer obtiendra une réponse avec un inhibiteur de point de contrôle immunitaire, comprenant les étapes consistant à i) isoler une population de cellules effectrices immunitaires d'un échantillon biologique obtenu auprès du patient, ii) mettre en contact la population de cellules effectrices immunitaires avec une population de cellules cibles pendant une période de temps suffisante et dans des conditions appropriées pour permettre à la population de cellules effectrices immunitaires d'induire une réponse cytotoxique en présence de l'inhibiteur de point de contrôle immunitaire, iii) mesurer le niveau de renouvellement de membrane réparatrice dans la population de cellules cibles en mesurant l'absorption d'un colorant lipophile fluorescent par la population de cellules cibles, iv) comparer le niveau de renouvellement de membrane réparatrice déterminé à l'étape iii) avec le niveau de renouvellement de membrane réparatrice mesuré en l'absence de l'inhibiteur de point de contrôle immunitaire et v) conclure le patient obtiendra une réponse avec l'inhibiteur de point de contrôle immunitaire lorsque le niveau de renouvellement de membrane réparatrice déterminé à l'étape iii) est supérieur au niveau de renouvellement de membrane réparatrice mesuré en l'absence de l'inhibiteur de point de contrôle immunitaire, dans lequel le colorant lipophile fluorescent est un colorant styryle.

20. Procédé selon la revendication 19 dans lequel le colorant styryle est choisi dans le groupe consistant en FM1-43, FM4-64, FM14-68, FM2-10, FM4-84, FM1-84, FM14-27, FM14-29, FM3-25, FM3-14, FM5-55, RH414, FM6-55, FM10-75, FM1-81, FM9-49, FM4-95, FM4-59 et FM9-40.

21. Procédé pour déterminer si un patient souffrant d'un cancer obtiendra une réponse avec un traitement comprenant l'étape consistant à i) mettre en oeuvre le procédé de la revendication 1 pour évaluer la capacité de CTL à fournir une réponse cytotoxique et la capacité des cellules tumorales à résister à la réponse cytotoxique ; b) mettre en oeuvre un algorithme sur des données comprenant les potentiels lytiques déterminés à l'étape i) de manière à obtenir une sortie d'algorithme ; c) déterminer la probabilité que le patient obtienne une réponse au traitement.
